# EUROPEAN PATENT APPLICATION

(11) **EP 4 686 477 A1**
(43) Date of publication of application: **04.02.2026**
(21) Application number: 24191599.0
(22) Date of filing: 29.07.2024
(51) Int. Cl.: A61K 31/713, A61P 25/02, C12N 15/113

(54) **COMPOUNDS AND COMPOSITIONS FOR TREATING PNS INJURY AND DEGENERATIVE DISEASES**

(71) Applicant: AdRegeneer, 4052 Basel (CH)
(72) Inventor: JACOB, Claire, 4052 BASEL (CH); HERTZOG, Nadège, 55122 Mainz Mainz (DE); DUMAN, Mert, 6006 Luzern (CH)
(74) Representative: Schneiter, Sorin

(57) **Abstract**

The present invention concerns and relates to compounds and compositions for treating injuries and or degenerative diseases of the PNS. The compounds and/or compositions that reduce intracellular levels of HDAC8 and/or short isoform TRAF7, prevent the binding of HDAC8 to short isoform TRAF7, and/or increase intracellular levels of long isoform TRAF7. In a preferred embodiment, the compounds and/or compositions include silencing RNAs that downregulate HDAC8 and/or short isoform TRAP7.

## Description

### Technical Field

The present invention generally relates to the field of treating injuries of the Peripheral Nervous System (PNS) as well as diseases affecting the health and function of the PNS. More specifically, the invention relates to compounds and composition that reduce intracellular levels of HDAC8 and/or short isoform TRAF7, prevent the binding of HDAC8 to short isoform TRAF7, and/or increase intracellular levels of long isoform TRAF7.

### Background Art and Problems Solved by the Invention

While the functions of the class I HDACs, HDAC1, HDAC2 and HDAC3, have been extensively studied in different areas of biology and medicine, the functions of HDAC8, the fourth and last member of the class I HDACs, have been comparatively less explored. It is however well established that HDAC8 mutations in humans affecting deacetylase activity can cause subtypes of Cornelia de Lange syndrome, a genetic developmental disorder affecting multiple functions (Sarogni, P., Pallotta, M. M. & Musio, A. Cornelia de Lange syndrome: from molecular diagnosis to therapeutic approach. J. Med. Genet. 57, 289-295 (2020)). This syndrome is due to a loss of function of the cohesin complex and HDAC8 has been shown to deacetylate SMC3, a subunit of the cohesin complex, which is required for recycling the complex for subsequent cell divisions. HDAC8 has also been involved in other pathological conditions including cancer, parasitic and viral infections, where it has mainly been shown to contribute to the pathology through its deacetylase activity (Kim, J. Y. et al. Pathological role of HDAC8: Cancer and beyond. Cells 11, 3161 (2022)).

WO2016149099 discloses α-cinnamide inhibitors of HDAC8 that are useful for the treatment of cancer and other diseases and disorders.

The functions of HDAC8 in the PNS remain however unknown (Gomez-Sanchez, et al, Emerging Role of HDACs in Regeneration and Ageing in the Peripheral Nervous System: Repair Schwann Cells as Pivotal Targets. Int. J. Mol. Sci. 2022, 23, 2996).

It is known that after a traumatic injury, the peripheral nervous system (PNS) can efficiently regenerate. This is to a large extent due to the high plasticity of Schwann cells (SCs), the myelinating glia and the main glial cell type of the PNS. Indeed, SCs react rapidly to a traumatic injury of the PNS by actively demyelinating and converting into repair cells that secrete neurotrophic factors which promote axonal regrowth, and form bands of Büngner that guide axons back to their former target. SCs then remyelinate or re-ensheath regenerated axons, which leads to successful functional recovery. In some cases, however, when the injury has created a large gap between axons and their target or in aged individuals, target re-innervation and functional recovery can partially or completely fail. Understanding the molecular mechanisms that control SC plasticity after injury has the potential to reveal drug targets for increasing SC plasticity and thereby accelerating axonal regrowth and remyelination efficiency.

Molecularly, the transcription factor c-Jun, which is rapidly upregulated in SCs after a PNS injury, is widely recognized as a master inducer of the SC demyelination process and conversion into repair cells, and is critical for axonal regrowth after injury. Interestingly, c-Jun expression levels in SCs after a PNS lesion are lower in aged individuals compared to young adults, and restoring c-Jun levels in aged individuals leads to axonal regeneration of similar efficiency as in young adults. Although the functions and target genes of c-Jun have been extensively studied in SCs after injury, the mechanisms that control c-Jun upregulation remain partially understood. Wagstaff et al. (Failures of nerve regeneration caused by aging or chronic denervation are rescued by restoring Schwann cell c-Jun. Elife 10, e62232 (2021)) showed an involvement of Sonic Hedgehog signaling in maintaining high c-Jun levels at 7 days post sciatic nerve cut lesion, but not at earlier time points. Our previous findings indicate that the regeneration process after a PNS injury is not optimal and can be improved. Other members than HDAC 1 and 2 of the HDAC family have also been described to be involved in the regeneration process of the PNS after injury. Indeed, HDAC3, another class I HDAC, has been reported to prevent precocious remyelination after lesion, while members of class II HDACs have been shown to be involved in the remyelination process.

A PNS lesion abruptly interrupts oxygen supply and therefore creates a hypoxic environment. Hypoxia is known as a strong inducer of c-Jun phosphorylation and upregulation. Indeed, hypoxia-inducible factor 1-alpha (HIF1a), a transcription factor that is rapidly targeted to the proteasome and degraded in the cell cytoplasm under normoxic conditions, is instead rapidly upregulated in hypoxic conditions by inhibition of its degradation. Under hypoxia, HIF1a upregulation can promote c-Jun-N-terminal kinases (JNK) activation and both HIF1a and phosphorylated JNK translocate to the cell nucleus, which leads to JNK-dependent c-Jun phosphorylation and to the activation of *c-Jun* transcription and c-Jun stabilization. The mechanisms regulating HIF1a stabilization and degradation have been extensively studied, particularly in cancer cells. However, they remain partially understood and little is known about HIF1a regulation in SCs.

KR102022155235A teaches miRNA Composition for preventing or treating neurodegenerative diseases. In one embodiment, KR102022155235A speculates that the miRNA-214-3p inhibitor acts on neuroinflammation through regulation of the expression of TRAF1, TRAF3, TRAF5, TRAF7, TBFSF10, CD27, and IKBKB, which are the targets to be bound to miRNA-214-3p.

Further objectives and problems addressed by the present invention will become apparent from the description of the aspects and embodiments of the invention herein below.

### Summary of the Invention

Remarkably, the present inventors provide novel treatments of all types of injuries or damages to the PNS, conditions affecting the PNS, and/or degenerative diseases of the PNS.

In an aspect, the invention provides a compound and/or a composition suitable to: reduce intracellular levels of HDAC8 and/or short TRAF7, prevent the binding of HDAC8 to short TRAF7, and/or increase intracellular levels of long TRAF7.

In a preferred embodiment, the compounds and/or compositions of the invention are for use in treatment and/or in a method of treatment, in particular of a human or animal subject suffering from a condition, including a disease or disorder.

In an aspect, the compounds and/or compositions of the invention are for use in the treatment of PNS injury and/or a degenerative disease of the PNS.

In some aspects, the compounds and/or compositions may be used for treating injuries of the PNS, damages to the PNS, diseases of the PNS, disorders and/or undesirable and/or damaging conditions of the PNS, in particular affecting the human or animal body.

In other aspects, the compounds and/or compositions may be used for treating injuries of the central nervous system (CNS), damages to the CNS, diseases of the CNS, disorders and/or undesirable and/or damaging conditions of the CNS, in particular affecting the human or animal body.

In another aspect, the invention provides a method for treating PNS injury and/or a degenerative disease of the PNS, the method comprising the step of administering, to an individual in need thereof, a therapeutically effective amount of a compound and/or composition according to the invention.

In an aspect, the compound and/or a composition of the invention is for use in one or more selected from: (1) treating damaged nerves of the PNS after injury or PNS damage other than due to injury, including degenerative disease, (2) promoting regeneration of PNS nerves after injury or PNS damage other than due to injury, (3) clearing myelin debris around damaged nerves of the PNS, (4) promoting the regrowth of axons of the PNS after injury or PNS damage other than due to injury, (5) promoting the recovery of nerves of the PNS after injury or PNS damage other than due to injury, (6) promoting sensory function recovery after PNS injury or PNS damage other than due to injury and/or, (7) promoting the conversion of Schwann cells into the repair phenotype.

In an aspect, the invention provides a method for one or more selected from the group consisting of any one or more of (1)-(7) as detailed above. The method comprising the step of administering, to an individual in need thereof, a therapeutically effective amount of a compound and/or composition of the invention.

In an aspect, the invention provides an isolated protein comprising the amino acid sequence of SEQ ID NO: 6, or an amino acid sequence having at least 80% sequence identity with SEQ ID NO: 6.

In a preferred embodiment, said protein comprises one or more selected from the group consisting of: a RING finger domain, E3 ubiquitin ligase domain and/or activity, a TRAF-type Zinc finger domain, and a coiled-coil domain. Preferably, said protein and/or said amino acid sequence comprises less than 4 complete WD40 repeat domains, preferably less than 3 or 2 WD40 repeat domains and most preferably only one truncated WD40 repeat domain or no complete or incomplete WD40 repeat domain.

In an aspect, the invention provides an isolated protein comprising, consisting essentially of and/or consisting of the amino acid sequence of SEQ ID NO: 6, or an amino acid sequence having at least 80% sequence identity with SEQ ID NO: 6.

In an aspect, the present invention provides an antibody or fragment thereof that specifically binds to short TRAF7 and/or the polypeptide defined by SEQ ID NO: 6, and/or the protein of the invention.

In an aspect, the invention provides an isolated protein comprising short TRAF7.

In an aspect, the invention provides a nucleotide sequence, such as a DNA or RNA sequence, encoding the protein of the invention, in particular short TRAF7, for example as disclosed in SEQ ID NO: 5.

In an aspect, the invention provides a method for screening for compounds and/or compositions potentially suitable in the treatment of injury to PNS nerves and/or for any of the pourposes reported in this specification, in particular for any one or more of the effects in (1) to (7) as recited in the context above with other aspects of the invention.

Further aspects and preferred embodiments of the invention are defined herein below and in the appended claims. Further features and advantages of the invention will become apparent to the skilled person from the description of the preferred embodiments given below.

### Brief Description of the Drawings

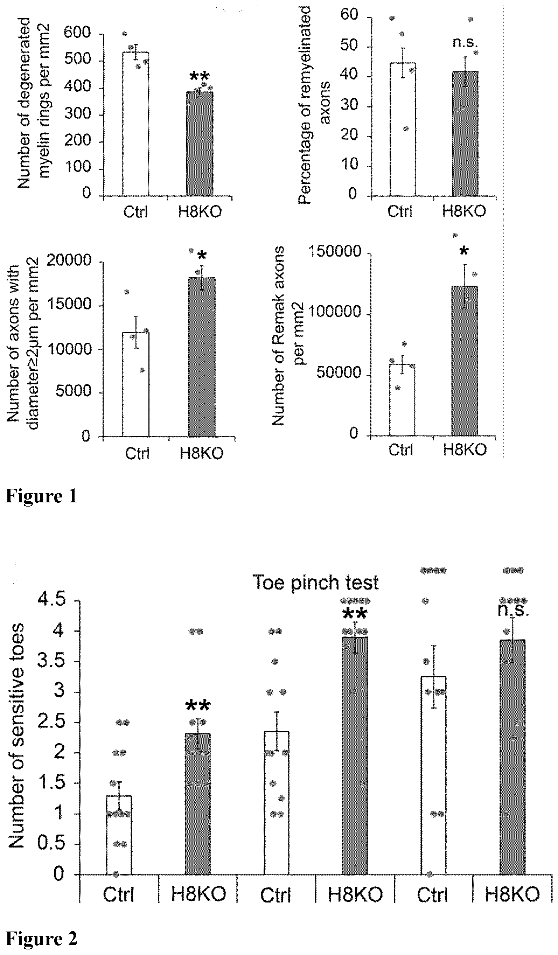

**Figure 1** shows graphs showing the number of degenerated myelin rings per mm², the percentage of remyelinated axons, the number of axons with diameter ≥ 2 µm per mm², and the number of Remak axons per mm² of HDAC8 KO (H8KO) and Control (Ctrl) sciatic nerves of adult mice at 12 dpl (days post lesion).
**Figures 2** shows a graph representing the performance of HDAC8 KO and Control mice at the Toe pinch test, n=12.

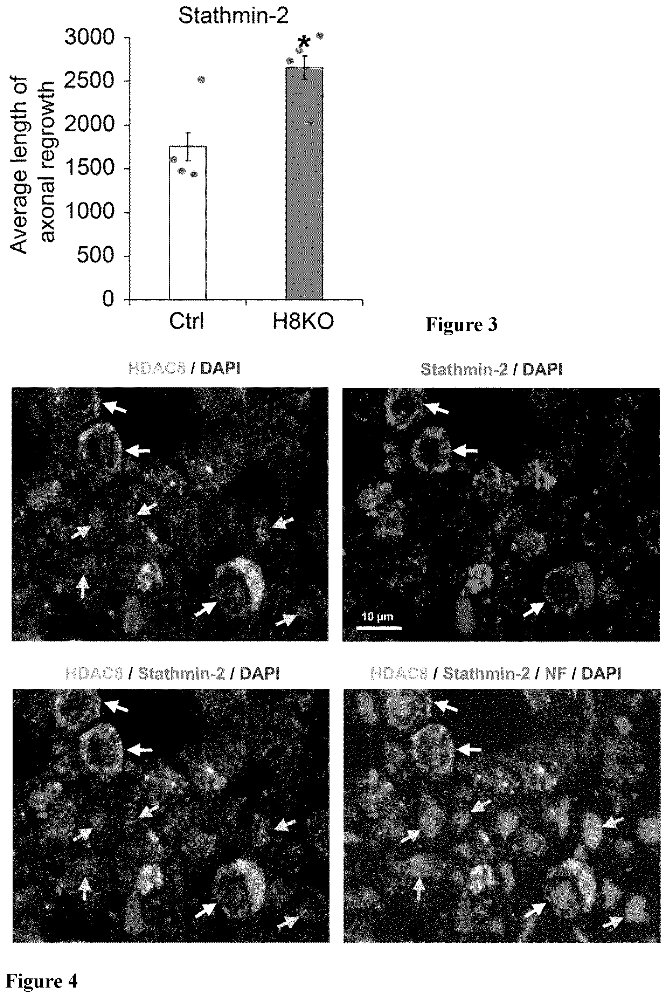

**Figure 3** shows quantification of axonal regrowth at 3 dpl showing longer regrowth in HDAC8 KO compared to Control nerves, n=4 animals per group, 45 to 76 regrowing axons measured and counted per animal.
**Figure 4** shows Co-immunofluorescence images of HDAC8 (green), Stathmin-2 (red) and Neurofilament (NF, magenta, general axonal marker) and DAPI labeling (blue) in a sciatic nerve cross-section of WT mice at 1 dpl. White arrows point to HDAC8-positive SCs surrounding Stathmin-2-positive axons, and blue arrows indicate HDAC8-positive axons (NF-positive).

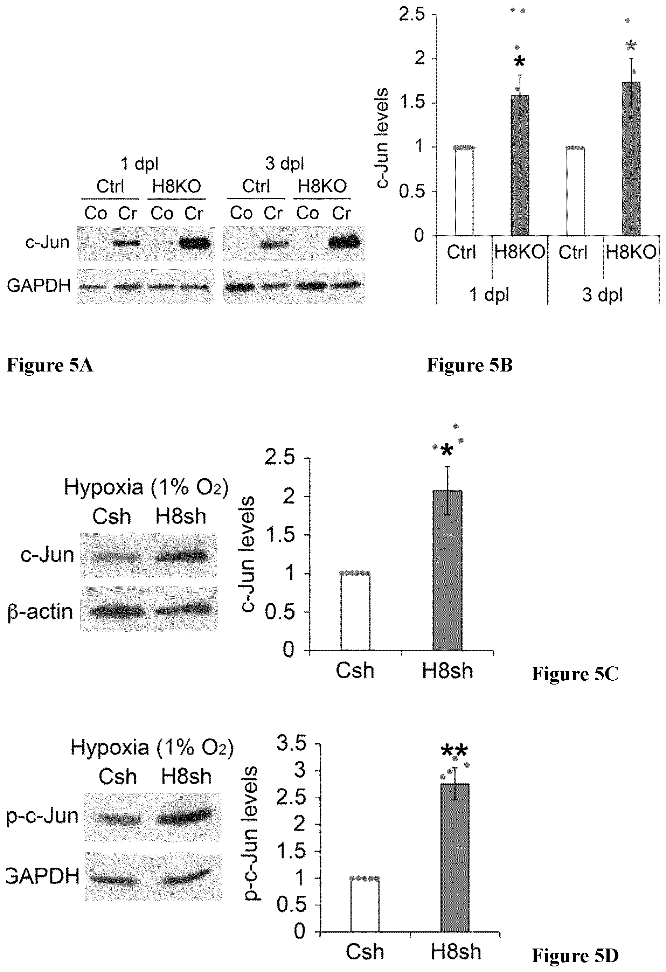

**Figures 5A** and **5B** show c-Jun Western blot (5A) and quantification (5B) normalized to GAPDH at 1 and 3 dpl, showing increased c-Jun levels in crushed sciatic nerves of HDAC8 KO mice compared to Control mice.
**Figures 5C** and **5D** show Western blots of c-Jun or phospho-c-Jun (left panels) in primary rat SCs cultured under conditions mimicking the conversion into the repair phenotype in hypoxia in hypoxic chamber for 16 h, and quantification normalized to GAPDH (right panels) showing that downregulation of HDAC8 by shRNA (H8sh) leads to increased c-Jun and phospho-c-Jun levels compared to cells incubated with a control shRNA (Csh).

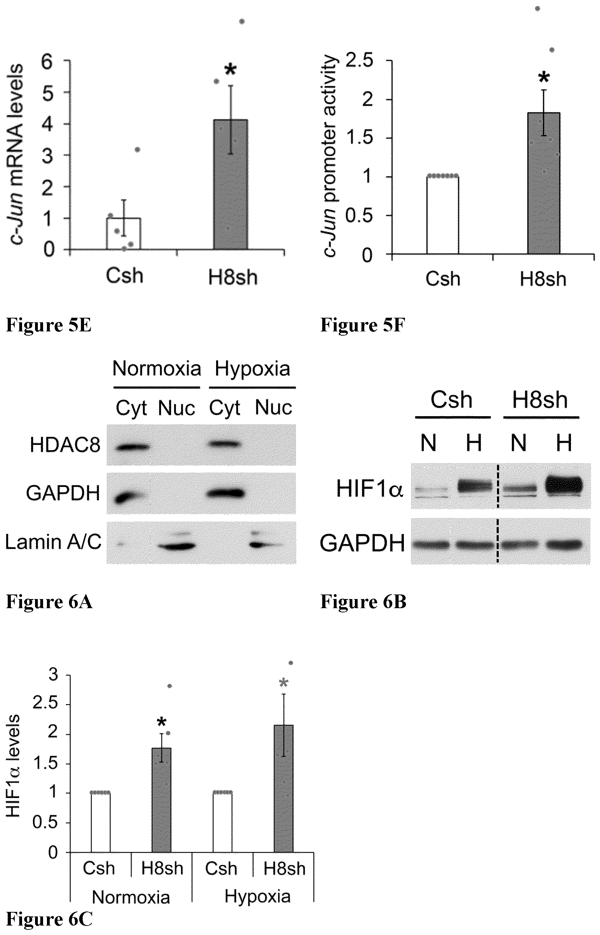

**Figures 5E** and **5F** show the quantification of *c-Jun* mRNA levels by qRT-PCR in primary rat SCs (Fig. 5E), showing increased *c-Jun* expression in cells where HDAC8 was downregulated by shRNA compared to cells incubated with a control shRNA, and the quantification of *c-Jun* promoter activity by luciferase gene reporter assay (Fig. 5F) in cells cultured as above, showing higher activity when HDAC8 was downregulated by shRNA compared to cells incubated with a control shRNA.
**Figure 6A** shows HDAC8 Western blot after subcellular fractionation of cytoplasmic (Cyt) and nuclear (Nuc) fractions of rat SCs cultured under normoxia or hypoxia (CoCl₂ for 16 h) in conditions mimicking the conversion into the repair phenotype. GAPDH and Lamin A/C, used as markers of the cytoplasmic and nuclear fractions, respectively, show fractionation efficiency.
**Figures 6B** and **6C** show HIF1α Western blot (Fig. 6B) on lysates of rat SCs cultured under the same conditions as above (Fig. 6A) in normoxia (N) or hypoxia (H) induced by CoCl₂ for 16 h, and quantification (Fig. 6C) normalized to GAPDH showing that the downregulation of HDAC8 by shRNA (H8sh) leads to increased HIF1α levels compared to cells incubated with a control shRNA (Csh).

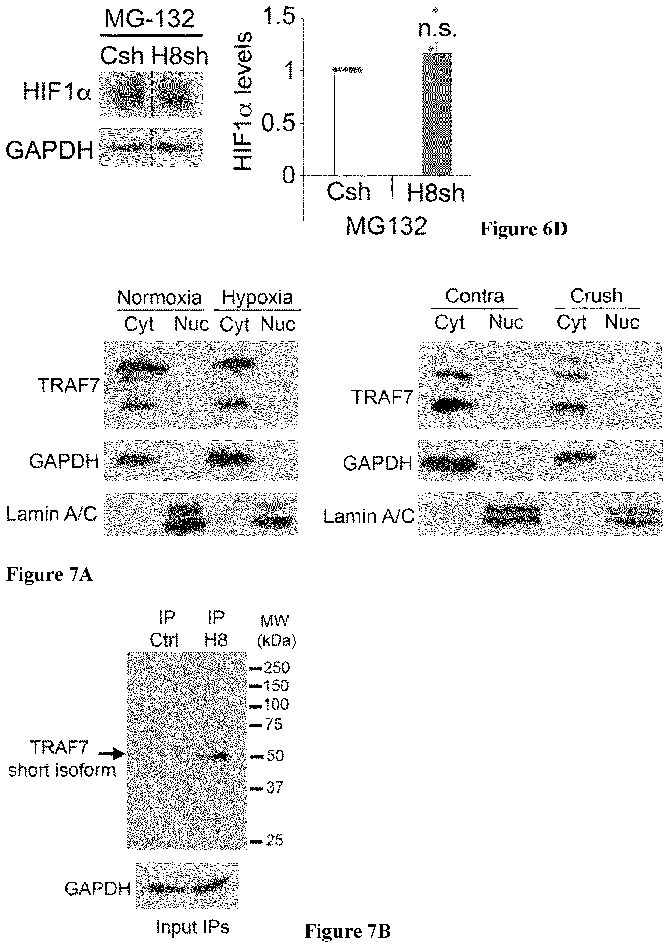

**Figure 6D** shows HIF1α Western blot (left panel) on lysates on rat SCs cultured under normoxia and incubated with the proteasome inhibitor MG-132 for 4 h, and quantification (right panel) normalized to GAPDH showing no significant difference (n.s.) of HIF1α levels in cells where HDAC8 was downregulated by shRNA (H8sh) compared to cells incubated with the control shRNA (Csh).
**Figure 7A** shows TRAF7 Western blot after subcellular fractionation of cytoplasmic (Cyt) and nuclear (Nuc) fractions of rat SCs cultured under normoxia or hypoxia (CoCl₂ for 16 h) in conditions mimicking the conversion into the repair phenotype (left panel) or crushed (Crush) and contralateral (Contra) mouse sciatic nerves at 1 dpl (right panel).
**Figure 7B** shows IP (Immunoprecipitations) HDAC8 (H8) or control IP (Ctrl, IgG) and Western blot of TRAF7 in lysates of rat SCs cultured in normoxia. The GAPDH input shows equal amount of lysates used for IP HDAC8 and control IP.

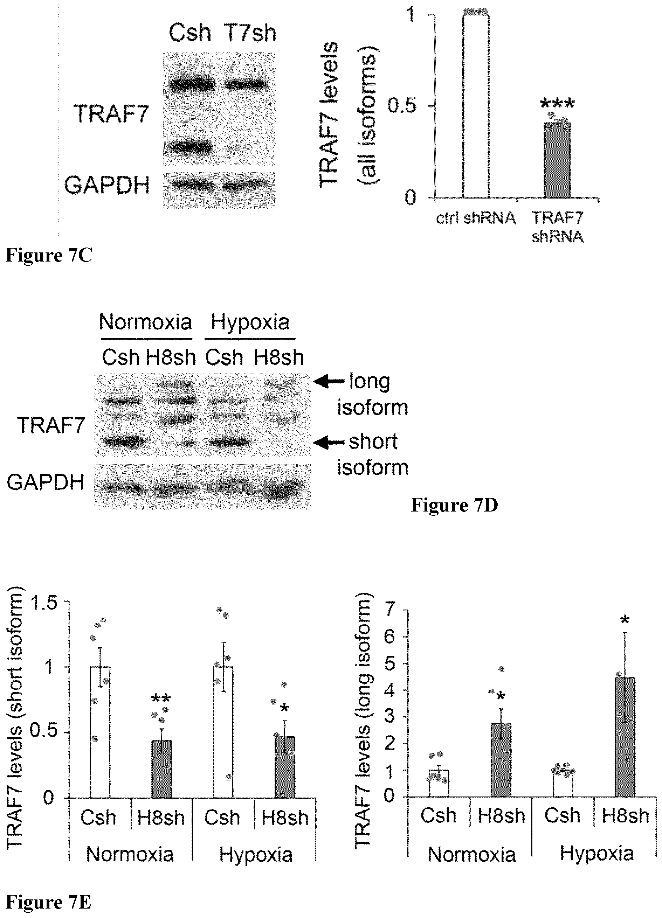

**Figure 7C** shows TRAF7 Western blot (left panel) in lysates of cells cultured in normoxia and hypoxia and incubated with lentiviruses carrying either an TRAF7 shRNA (T7sh) or a control shRNA (Csh), and quantification normalized to GAPDH (right panel) showing decreased levels of TRAF7 (all isoforms) in cells where TRAF7 was downregulated.
**Figures 7D** and **7E** show TRAF7 Western blot (Fig. 7D) in lysates of cells cultured in normoxia and hypoxia and incubated with lentiviruses carrying either an HDAC8 shRNA (H8sh) or a control shRNA (Csh), and quantification (Fig. 7E) normalized to GAPDH showing decreased levels of the short TRAF7 and increased levels of the longest TRAF7 isoform in cells where HDAC8 was downregulated.

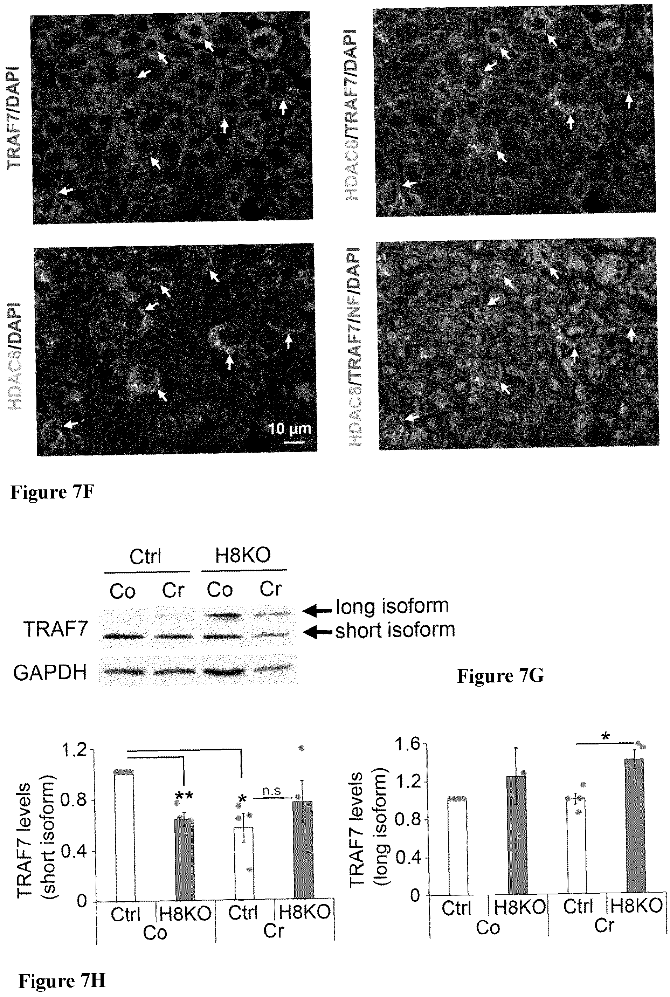

**Figures 7F** Co-immunofluorescence images (z-series projection of confocal stacks) of TRAF7 (red), HDAC8 (green) and Neurofilament (NF, magenta, axonal marker), and DAPI labeling (blue, nuclei) in cross-section of adult mouse sciatic nerve showing TRAF7 expression in all SCs (rings surrounding axons).
**Figures 7G** and **7H** show TRAF7 Western blot (Fig. 7G) in lysates of HDAC8 KO (H8KO) and control (Ctrl) crushed (Cr) and contralateral (Co) mouse sciatic nerves at 1 dpl, and quantification normalized to GAPDH (Fig. 7H) showing decreased levels of the short TRAF7 and increased levels of long TRAF7 in H8KO nerves.

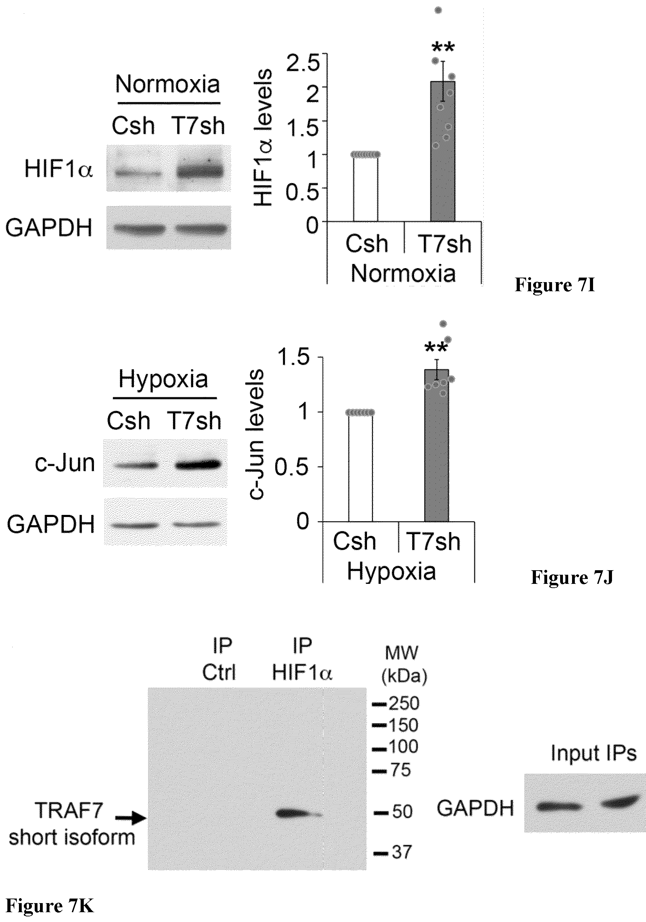

**Figures 7I** and **7J** show Western blot of HIF1α (Fig. 7I) or c-Jun (Fig. 7J) (left panels) in lysates of rat SCs cultured in normoxia (Fig. 7I) or hypoxia (Fig. 7J) and incubated with lentiviruses carrying a TRAF7 shRNA (T7sh) or a control shRNA (Csh), and quantification (right panels) normalized to GAPDH showing increased levels of HIF1α and c-Jun in cells where TRAF7 was downregulated.
**Figures 7K** shows IP HIF1α or control IP (Ctrl, Flag antibody) and Western blot of TRAF7 in lysates of rat SCs cultured in normoxia and incubated for 8 h with the proteasome inhibitor MG-132.

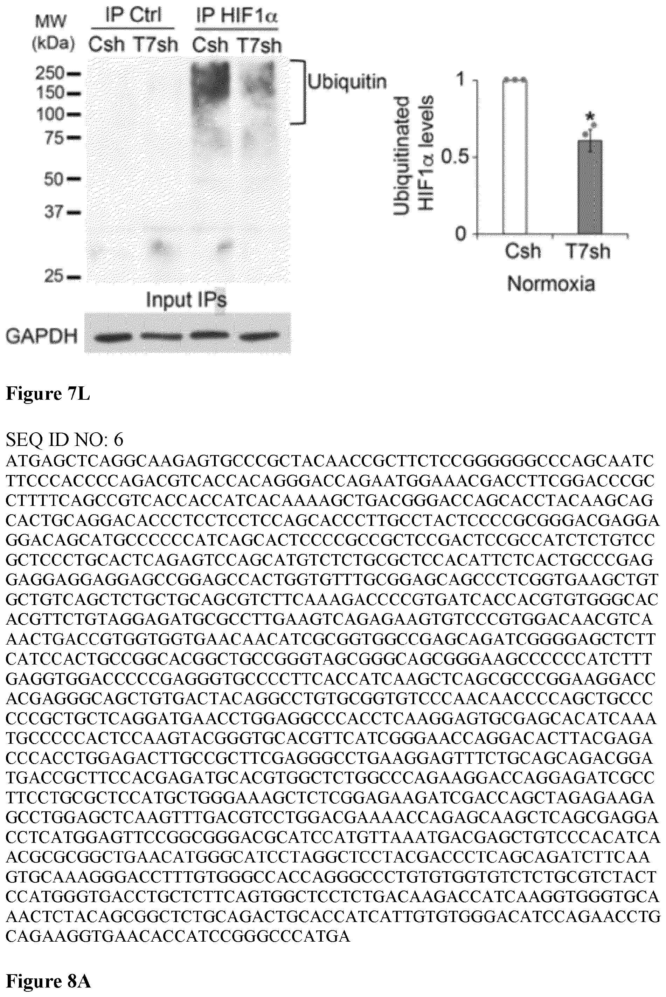

**Figures 7L** shows denaturing IP of HIF1α and control IP (Ctrl, GFP antibody) and Western blot of ubiquitin (P4D1) in lysates of SCs transduced with TRAF7 shRNA (T7sh) or control shRNA (Csh) lentivirus and cultured in normoxia and incubated for 8 h with MG-132, and quantification of ubiquitinated HIF1α levels in lysates of cells transduced with T7sh lentivirus compared to Csh lentivirus.

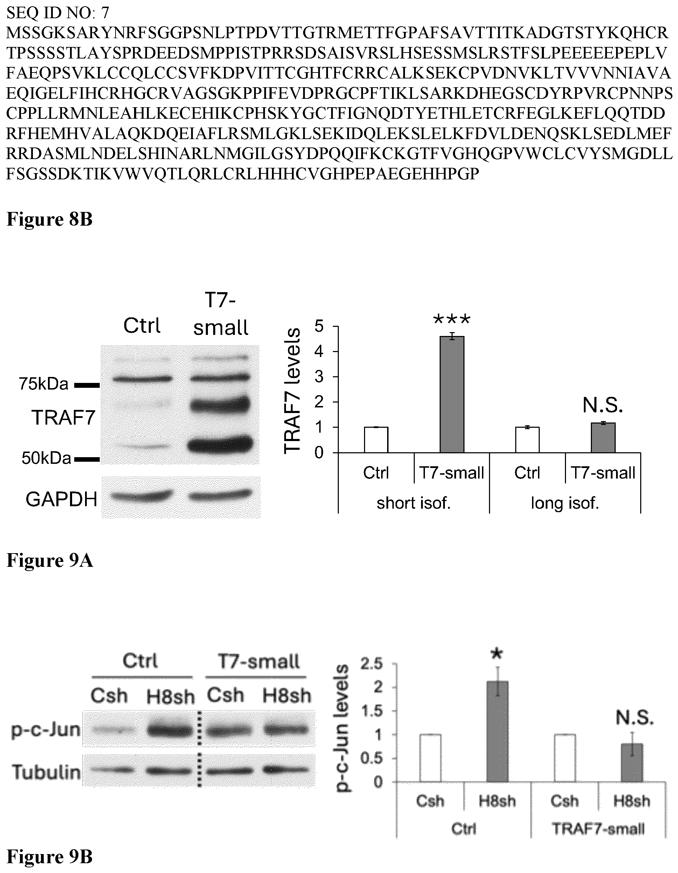

**Figures 8A** and **8B** shows nucleotide (Fig. 8A) and amino acid sequences (Fig. 8B) of short TRAF7 that is described herein (SEQ ID NOs: 5 and 6).
**Figure 9A** shows TRAF7 Western blot (left panel) on SCs transfected with a construct expressing short TRAF7 and quantification normalized to GAPDH (right panel).
**Figure 9B** shows Western blots of phospho-c-Jun (p-c-Jun) on lysates of rat SCs incubated with lentiviruses carrying a HDAC8 shRNA (H8sh) or a control shRNA (Csh) and subsequently transfected with a short TRAF7-overexpressing construct or a Flag-expressing construct as control (Ctrl), and quantification normalized to Tubulin showing that TRAF7 overexpression by transfection reverts the increase of p-c-Jun levels mediated by HDAC8 downregulation.

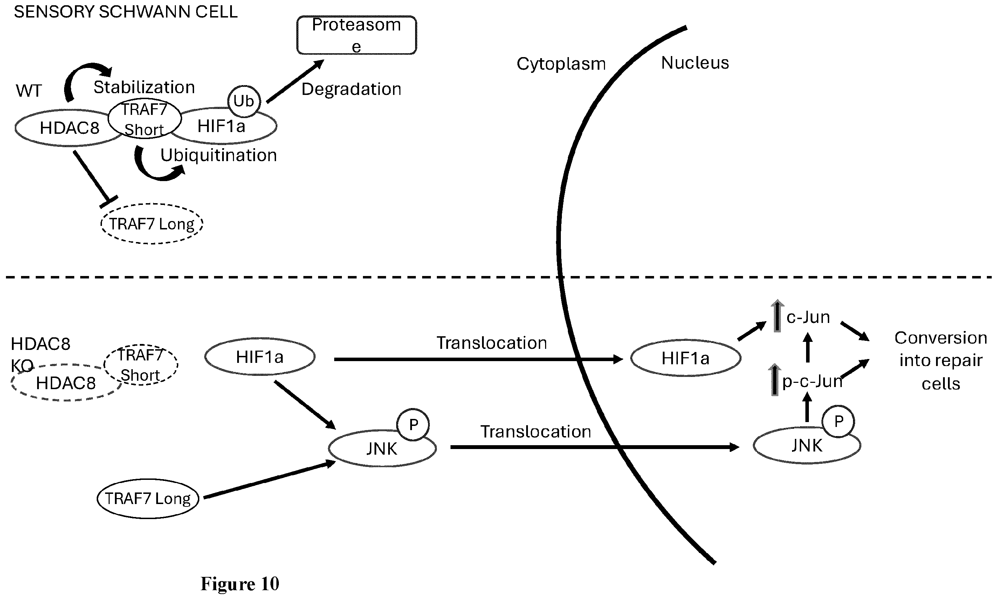

**Figure 10** shows the mechanism of action of HDAC8 in SCs after injury, in accordance with the findings of the present invention.

Hereinafter, preferred embodiments of the device of the invention are described, in order to illustrate the invention, without any intention to limit the scope of the present invention.

### Detailed Description of the Preferred Embodiments

The present invention relates to compounds and/or a compositions that have a biological activity selected from the group consisting of: reducing intracellular levels of HDAC8 and/or short TRAF7, preventing the binding of HDAC8 to short TRAF7, and/or increasing intracellular levels of long TRAF7.

In this specification, the expression "short TRAF7" is used to refer to "the short isoform of TRAF7" or "the short isoform TRAF7". The expression "long TRAF7" is used to refer to "long isoforms of TRAF7" or "long isoform TRAF7". Repetition of the word "isoform" is thus reduced, but used depending on context. "Short TRAF7" and "long TRAF7" are defined and/or characterized in more detail elsewhere in this specification, in particular in terms of structure and/or function.

Preferably, the compound comprises or is an active principle, i.e. an Active Pharmaceutical Ingredient (API). The compound may be any type of compound and/or molecule suitable to achieve the desired effects preferably at a molecular level. Preferably, the compound functions as an active principle exerting one or more of the biological effects mentioned in this specification. In some embodiments, the compound is a small molecule, in particular a small chemical molecule, which is understood herein as a compound having a molecular weight of ≤ 1000 daltons. For example, a small molecule may bind to HDAC8 and prevent it from binding to short TRAF7, or, may bind to short TRAF7 and prevent it from binding to HDAC8. Oligomers, such as oligopeptides, -saccharides, -nucleotides, or other oligomeric compounds are generally but need not necessarily be small molecules according to this definition. Oligomers include compounds that include from 2-10 units of a similar or same compound class or type, where the units may be identical or having a different structure but belong to the same class of compounds.

The compound, be it a small molecule or a larger molecule, may also be a compound that indirectly affects the levels of HDAC8 and/or TRAF7, for example by enhancing the effect of a suppressor of HDAC8 and/or short TRAF7, and/or by reducing the effects of a promotor of HDAC8 and/or short TRAF7.

In other embodiments, the compound is or comprises a larger molecule (>1000 daltons) such as a polymer, including a polypeptide or a protein comprising one or more polypeptides, a polysaccharide, a polynucleotide and/or any other polymeric molecule, including oligo- and polynucleotides, and nucleic acids, such as single strand and double strand polynucleotides and/or nucleic acids, including RNA (ribonucleic acid) and DNA (deoxyribonucleic acid) molecules. Polymers preferably are compounds comprising more than 10 units, which may be the same or different structure, but generally from the same compound class, such as a polynucleotide which is generally composed of a plurality of nucleotides that are selected from a limited number of available nucleotides, in particular the nucleotides comprising guanine, adenine, cytosine, and thymine or uracil, making up DNA and RNA.

In some embodiments, the compound and/or composition comprises an interfering oligo- or polynucleotide, for example operating by RNA interference (RNAi) or RNA silencing (RNAsi), such as small RNA molecules, for example one or more selected from short (or small) hairpin RNA (shRNA), microRNA (miRNA), small interfering RNA (siRNA), piwi-interacting RNA (piRNA), or others. In RNAi, double-stranded RNA (dsRNA) is generally used. In other embodiments, the compound comprises antisense oligonucleotides (ASOs) and the treatment involves antisense therapy. In ASO, single-stranded synthetic nucleic acids are generally used.

In an embodiment, said compound and/or composition is or comprises interfering RNA (RNAi), such as a short hairpin RNA (shRNA) or an siRNA, for example an siRNA duplex. ShRNA and siRNA duplex molecules can be designed by the person skilled in the art once the sequence of the gene or isoform to be silenced is known. The shRNA and siRNA duplex molecules comprise a targeting sequence, which is present in cDNA of the organism in question.

ShRNA sequences comprise a targeting sequence, a complementary sequence (to the targeting sequence) and further comprising a hairpin loop nucleotide sequence connecting the targeting and complementary sequences. The targeting sequence of a shRNA is preferably 18-30, but generally 19-29, preferably 19-25 nucelotides long. Generally, the hairpin loop is at the 3' end of the targeting sequence and has a length of 4-15 nucleotides. The shRNA sequence may comprise additional sequences and/or nucleotides, such as a basepair mismatch at the 5'-and/or 3'-ends of the hairpin sequence, a terminator and/or an overhang for inserting a restriction enzyme site (depending on the vector and/or plasmid used). The shRNA sequence can be prepared once a targeting sequence is identified, as the other sequences are known or can be designed.

SiRNA duplex molecules lack a hairpin and comprise the targeting sequence, the complementary sequence and generally an overhang sequence at the 3'- or 5'-end of each of the targeting and complementary sequences. The targeting sequence in a siRNA duplex generally has 18-24, but generally 19-23, preferably 19-21 nucelotides. The overhand sequences have, independently, from 1-10, preferably 2-5, most preferably 2-4 nucleotides. Exemplary overhangs are dTdT, GU or AU (or any RNA di-nucleotide) overhangs. Many other overhangs can be conceived or used, and are added at the 3'- or 5'-ends of the RNA of the targeting and complementary sequences. The two overhand sequences need not be identical in a siRNA duplex.

Examples of targeting sequence for siRNA or shRNA for human HDAC8 (custom designed) are GGGAAGGGATGGTACTACAGT (SEQ ID NO: 1) and GTGAAAGCGTACTAAAGGAAG (SEQ ID NO: 2).

An exemplary shRNA sequence (DNA) using the targeting sequence (SEQ ID NO: 1) for addition in a plasmid or vector would be:
TGGGAAGGGATGGTACTACA*TTCAAGAGA*TGTAGTACCATCCCTTCCCTTTTTTC (SEQ ID NO: 7, forward oliugonucleotide). The reverse nucleotide sequence would be:

TCGAGAAAAAAGGGAAGGGATGGTACTACGTCTCTTGAACGTAGTACCATCCCTT
CCCA (SEQ ID NO: 8).

Starting from 5'- end, the forward nucleotide (SEQ ID NO: 7) comprises a T nucleotide for generating a restriction enzyme cutting site (here HpaI) overhang for cloning into plasmid (in bold above), following by the targeting sequence, the hairpin loop sequence (TTCAAGAGA, shown italic), the complementary sequence, a terminator sequence (TTTTTT, underlined dotted), and a C (bold) nucleotide which contributes to forming the second restriction site (XhoI). In the reverse nucleotide (SEQ ID NO 8), the restriction site complement starts with the motif TCGAG at the 5' (in bold) end to complement the second restriction site (XhoI) and finishes with an A nucleotide (in bold) to complement the first restriction site (HpaI). The isolated, underlined G and C residues flanking the loop are mismatches added for increasing the efficiency of the molecule.

Examples of targeting sequences for siRNA or shRNA for human short TRAF7 (custom designed) are GGTGGGTGCAAACTCTACAGC (SEQ ID NO: 3), GACCATCAAGGTGGGTGCAAA and (SEQ ID NO: 4).

Targeting sequences are generally indicated as DNA sequences, even though they may be administered as RNA molecules, for example in case of siRNA duplex.

If the compound of the invention comprises an oligo- or polynucleotide, such as an interfering RNA (e.g. shRNA), it may be associated with and/or integrated in a delivery vehicle, such as a plasmid, or any viral or bacterial vector. A variety of viral vectors can be used to obtain shRNA expression in cells, including adeno-associated viruses, adenoviruses, and lentiviruses, for example.

In some embodiments, the compound, e.g. the oligo- or polynucleotide, such as an interfering RNA (e.g. shRNA), is associated with and/or integrated in a particle, such as a nanoparticle, including liposomes or protein-based nanoparticles. The particle may be targeted to specific cells, in particular Schwann cells. To this end, the particle may carry a targeting molecule on its surface, for example.

In some embodiments, the compopund, e.g. oligo- or polynucleotide, such as an interfering RNA (e.g. shRNA), is covalently connected to a molecule comprising a lipophilic component, such as a cholesterol molecule, which may help the interfering RNA to pass the membrane of the target cell, in particular the Schwann cell.

In an embodiment, said compound and/or composition is or comprises a repressor of HDAC8 or an oligo- and/or polynucleotide sequence encoding a repressor of HDAC8.

More generally, the compounds of the invention may comprise and/or consist essentially of a plurality of compounds/parts from different compound classes, wherein the different parts provide different functionalities to the overall compound. Typically, the compound comprises an interfering nucleotide such as an shRNA, for example, a compound that facilitates entry into the cell, such as a compound comprising a lipophilic part (e.g. cholesterol), and optionally a targeting compound, which specifically binds to and/or is taken into cells of a particular cell type.

The invention encompasses compounds and/or compositions for treatment. The compositions may comprise the compound of the invention. In an embodiment, the composition is a pharmaceutical composition. The composition may be in a solid or liquid form. The pharmaceutical composition may comprise an active principle, such as the compound of the invention, and one or more additional compounds and/or ingredients, such as a pharmaceutically acceptable carrier and/or excipient. A pharmaceutically acceptable carrier is preferably selected depending on the administration form, such as solid or liquid. Pharmaceutically acceptable carriers may include one or more selected from surfactants, polymers, lipids (such as fatty acids and liposomes), and solvents. Polymers may include natural (e.g. sodium alginate, gelatin, chitosan), semi-synthetic (e.g. cellulose derivatives, such as alkyl and acetyl cellulose), and synthetic polymers (e.g. polyethylene glycols, poloxamers, polyactides, polyamides, acrylic acid polymers), and fermentation products (e.g. xantham gum). Solvents preferably include organic solvents, alcohols and water, preferably sterile water. In preferred embodiments, the pharmaceutical composition is an injectable solution and/or composition, preferably a sterile injectable solution, suspension and/or emulsion, which preferably comprises water.

The pharmaceutical composition preferably comprises a therapeutically effective amount of the compound. A therapeutically effective amount is the dose or concentration of a drug that produces a desired biological response. The therapeutically effective amount is an amount that produces a clinically significant response in average efficacy, which is also statistically significantly superior to the response provided in placebo. A therapeutically effective amount is generally suitable to obtain a concentration at the target site which is in the therapeutic range, wherein the latter is determined by the minimum effective dose (MED) and the maximum tolerated dose (MTD), for example according to pharmacological norms established preferably at the date of the filing of the present application. The MED and/or MTD typically depend on the patient population and the individual to be treated (e.g. human or animal). Preferably, these limits apply to the average patient population. Generally, the MED is above a dose that is obtained by normal, average or even excessive consumption of food and/or drinks that may comprise compounds that exhibit the biological activity that is desired in accordance with the present invention.

The composition is preferably selected and/or determined depending on the form of administration. The present invention is not generally limited to a form of aministration. In some embodiments, the compound and/or composition is systemically administered.

Exemplary forms of administration in accordance with the invention include oral, sublingual, buccal, nasal, rectal, parenteral, intravenous, subcutaneous, intramuscular, intraarterial, transnasal, intranasal, vaginal, transdermal, inhalational and intraosseous administration.

In a preferred embodiment, the compound and/or composition is administered locally and/or at or in proximity of the site of the injury and/or lesion of the nerves and/or neurons to be treated. Accordingly, preferred forms of administration include transdermal and/or subcutaneous administration. In a preferred embodiment, said compound and/or composition is administered by transdermal administration and/or in the form of a subcutaneous depot, preferably at the site of injury, lesion and/or at the site of the damaged nerve or neuron of the PNS.

In some embodiments, the invention encompasses administering the compound and/or composition to an individual in need of a treatment. The individual may be a human or an animal. The animal may be a domestic animal, for example. More generally, the animal to be treated may be poultry, fish, a mammal animal, e.g. livestock, domestic animal, or pet, such as a dog, cat, rodent, guinea pig, mouse, for example.

It is noted that if the treatment involves RNAsi, and/or the compound comprises a nucleotide, such as a shRNA or any other oligo- or polynucleotide mentioned in this specification (wherein the oligo- or polynucleotide is intended for binding to a complementary nucleotide sequence of the individual to be treated), the said nucleotide is preferably specific to the species of the patient to be treated. For example if the patient is human, the shRNA or siRNA is specific to the human complementary mRNA that is the target of the shRNA or siRNA.

Similarly, reference to HDAC8 and TRAF7 in the context of the present invention is generally reference to the corresponding HDAC8 and TRAF7 of the species to be treated, except where the species is specifically indicated to be different. In some embodiments, the treatment is for humans, such that reference to HDAC8 and TRAF7 is reference to human HDAC8 and TRAF7. If the individual to be treated is an animal, the reference to HDAC8 and TRAF7 is reference to the HDAC8 and TRAF7 of the corresponding (e.g. dog, cat, etc) animal.

The compound and/or composition of the invention preferably exert a biological activity, which defines them as active principle or API. The biological activity is preferably an activity that takes place at a molecular level, preferably within cells, and most preferably in the cytoplasm. Accordingly, the pharmaceutical composition and/or compound is preferably suitable for reaching the cytoplasmic compartment and preferably the cytoplasm of cells, preferably Schwann cells. In some embodiments, the compound and/or pharmaceutical composition comprises or is associated with a suitable delivery vehicle, as exemplified elsewhere in this specification.

The compound and/or composition preferably exhibits one or more of the following biological activities:
1. The compound and/or composition reduces the intracellular levels of HDAC8 and/or short TRAF7,
2. The compound and/or composition prevents the binding of HDAC8 to short TRAF7, and/or,
3. The compound and/or composition increases the intracellular levels of long TRAF7.

In an embodiment, the above mentioned reduction of HDAC8 and/or short TRAF7, and/or prevention or binding of HDAC8 to short TRAF7, and/or, the increase of intracellular levels of long TRAF7 is and/or takes place in Schwann cells (SCs), preferably in the cytoplasm of said SCs.

The compounds and/or compositions preferably achieve these effects *in vivo*, and/or within the cytoplasm, in particular of said Schwann cells, while leaving said cells intact, alive and/or preferably enabling their conversion into, maintaining, and/or stabilizing the repair phenotype of the Schwann cells.

HDAC8 is Histone deacetylase 8, also referred to herein as H8. TRAF7 is TNF receptor associated factor 7, also referred to as T7 herein and/or in the figures. Preferably, the HDAC8 and TRAF7 porteins and/or isoforms as disclosed herein are all naturally occurring proteins and/or isoforms. In some embodiments, the TRAF7 and/or HDAC8 proteins and/or isoforms may be isolated and/or purified, for example when used in a screening method in accordance with the invention. In some embodiments, the TRAF7 and/or HDAC8 proteins and/or isoforms are recombinant, for example when produced for and/or used in the screening method of the invention.

HDAC8, in case of human HDAC8, has the previously reported mRNA and amino acid sequence as shown in GenBank accession number AJ277724, described by Van den Wyngaert, I. et al, Cloning and characterization of human histone deacetylase 8, FEBS Lett. 478 (1-2), 77-83 (2000).

Preferably, said HDAC8 referred to in the context of treatment is a naturally occurring HDAC8, preferably full-length HDAC8.

In an embodiment, said short TRAF7 includes TRAF7 isoforms that have a molecular weight of less than 70kDa, less than 68kDa, less than 67kDa, less that 65kDa, preferably less than 60kDa, even more preferably less than 55kDa, and most preferably about 50kDa, for example from 50-75kDa, 53-70kDa, or 54-56kDa.

Preferably, said short TRAF7 referred to in the context of treatment is a naturally occurring isoform.

In an embodiment, said short TRAF7 is the isoform encoded by the nucleotide sequence of SEQ ID NO: 5.

In an embodiment, said short TRAF7 is an isoform comprising a RING finger domain, E3 ubiquitin ligase activity, TRAF-type Zinc finger domain, a coiled-coil domain, and less than 4 complete WD40 repeat domains, preferably less than 3 or 2 WD40 repeat domains and most preferably only one truncated WD40 repeat domain or lacking any amino acid sequence belonging to a WD40 repeat domain.

In an embodiment, long TRAF7 is and/or includes TRAF7 isoforms that have a molecular weight of higher than 55kDa, preferably higher than 60kDa, and more preferably about 65kDa or higher, most preferably 68kDa or 70kDa or higher, for example isoforms having molecular weights of up to about 75kDa, for example up to 80kDa.

In an embodiment, long TRAF7 is and/or includes the isoform encoded by any one of the nucleotide sequences disclosed under accession numbers NM_032271, XM_005255627.6, XM_011522700.2, and XM_054314158.1.

In an embodiment, long TRAF7 is and/or includes isoforms comprising a RING finger domain, E3 ubiquitin ligase domain and/or activity, TRAF-type Zinc finger domain, a coiled-coil domain, and 4 or more, 5, 6 or more, most preferably 7 complete WD40 repeat domains. Preferably said long TRAF7 referred to in the treatment is naturally occurring.

Preferably, said long TRAF7 referred to in the context of treatment is a naturally occurring isoform, preferably a naturally occurring full-length TRAF7 isoform.

In the context of the present invention, the terms "reducing intracellular levels" and "decreasing intracellular levels" refers to the increase or reduction, respectively, of any one selected from the concentration, the occurrence, the abundance, the number, the quantity, of the protein referred to, in particular in the cytoplasm. Preferably, said increase and/or reduction is a measurable increase and/or decrease, and is preferably determined in comparison with cells that were not treated and/or exposed to the compounds having the activities referred to herein. For example, said increase and/or decrease is preferably determined in comparison with individuals and/or cells that have received a control treatment, no treatment and/or a placebo.

The increase and/or decrease referred to in this specification may be obtained by any molecular known or unknown mechanism.

In a preferred embodiment, the compound and/or a composition of the invention is capable of reducing intracellular levels of HDAC8 and/or short TRAF7 by one or more selected from:
(a) repression of gene activation and/or expression of HDAC8 and/or short TRAF7,
(b) downregulation of HDAC8 and/or short TRAF7,
(c) otherwise reduction of intracellular levels of HDAC8 and/or short TRAF7.

The mechanisms of repression and downregulation are well known to the person skilled in the art, and the use of RNAi for downregulation is exemplified in the present specification. Other ways of downregulation are also encompassed, including gene editing, such as editing, e.g. mutating, deleting and/or truncating the gene encoding HDAC8, in particular in Schwann cells, for example by CRISPR/Cas, the use of small molecule effectors and/or miRNA that downregulates HDAC8. It is noted that said downregulation is preferably specific, such that the expression of other genes is preferably not affected or not significantly affected, or if affected, does not have any or therapeutically relevant effect.

Repression may include switching off the gene encoding HDAC8, for example by epigenetic mechanisms. In the context of the present specification, "repression" includes the use of DNA- or RNA-binding proteins that inhibit the expression of one or more genes (here: of the gene/RNA encoding HDAC8 or the gene/RNA encoding short or long TRAF7), in particular by binding to the promoter, enhancers or associated silencers. A DNA-binding repressor blocks the attachment of RNA polymerase to the promoter of a gene of interest (e.g. encoding HDAC8 or short TRAF7) thus preventing transcription of the gene into messenger RNA.

In a preferred embodiment, the compound and/or a composition of the invention is capable of increasing intracellular levels of long TRAF7 by one or more selected from:
(d) upregulation of long TRAF7,
(e) inducing the expression of long TRAF7, and/or
(f) otherwise increase intracellular levels of long TRAF7.

Similar to gene repression, the concept of inducers and inducing gene expression are known in the art. In the context of the invention, repression and/or induction may be obtained by administering directly a repressor and/or inducer protein, or by administering a vector or plasmid comprising a nucleotide sequence encoding a repressor or inducer protein, for example.

"Preventing of binding of HDAC8 to short TRAF7" may be achieved by administering a compound that binds to one or both selected from of HDAC8 and short TRAF7 and thereby prevents HDAC8 to bind to short TRAF7, for example by physically preventing the two proteins from their binding interaction by occupying the site of binding, or by inducing a conformational change in any one of HDAC8 and/or TRAF7, thereby reducing the binding affinity of one for the other. The compound may be or comprise a small molecule, an oligo- or polypeptide, or any other molecule class. The compound may also result in the targeting of HDAC8 and/or TRAF7 to the proteasome, for example, or induce degradation of any one of the two proteins in other ways.

In a preferred embodiment, the compound and/or composition does not substantially affect, preferably not substantially, inhibit, the enzymatic deacetylase activity of HDAC8. If the compound and/or composition exerts the biological activity specified herein, it is generally not relevant if the compound also inhibits the deacetylase activity, as long as the inhibitory activity is found in addition to the biological activity as specified herein. Preferably, said compound and/or composition is not an inhibitor of HDAC8 enzymatic activity. As the present inventors surprisingly found, the beneficial effects reported herein are preferably obtained by preventing the formation of a binding complex including HDAC8 and at least short TRAF7 and is not dependent on the enzymatic activity of HDAC8. Inhibitors of HDAC8 alone are not likely and generally will not achieve the beneficial effects and activities disclosed herein, unless such inhibitors reduce intracellular levels of HDAC8, which is generally not the case, especially in case of inhibitors that block the active site of HDAC8, or that bind to any unrelated site and may affect the conformation of HDAC8, without affecting its capacity to bind to shortTRAF7.

The present inventors have surprisingly found that compounds and/or compositions exhibiting the biological activities identified in this specification are suitable in treatment and/or prophylaxis, including methods of treatment. In particular, the compounds and/or compositions may be used for treating a condition, in particular a medical condition, where a "condition" is a defective, insufficient, undesired, unhealthy and/or uncomfortable state of health and/or being.

In particular embodiments, the condition involves damaged, injured, traumatized, impaired and/or otherwise unfuctional nerves, neurons and/or ganglia and/or parts of the nervous system, for example nerves and/or neurons comprising a physical lesion. The nerve and/or neuron is preferably a nerve of the peripheral nervous system (PNS), but the invention may possibly and is even likely to also work with nerves and/or neurons of the central nervous system (CNS). The damages of nerves, neurons and/or ganglia may have any origin, such as exernal causes, in particular due to impacts and/or physical injuries, but also other causes than due to injury, such as PNS damage caused and/or associated with diseases and/or disorders due to genetic causes, autoimmune diseases, chronic or acute inflammation, infections, such as viral and bacterial infections, but also due to normal processes such as increasing age, and so forth, for example.

In a preferred embodiment, said PNS is specifically the somatic nervous system of the PNS. The expression "specifically the somatic nervous system" is intended to mean that the treatment relates and/or is specific to nerves, neurons and/or glia of the somatic nervous system but not to other nerves or neurons, such as those of the visceral nervous system. For example, the treatment may be suitable to repair damages and/or injuries of the somatic nervous system but not of the visceral nervous system.

In a preferred embodiment, said PNS is specifically sensory PNS and/or nerves and/or neurons of said PNS are specifically sensory nerves. The expression "specifically the sensory nervous system" is intended to mean that the treatment relates and/or is specific to nerves, neurons and/or glia of the sensory nervous system but not to other nerves, such as those of the motor nervous system and/or motor neurons of the PNS and optionally also of neurons of the visceral PNS. For example, the treatment may be suitable to repair damages and/or injuries of sensory nerves but not of motor nerves, preferably of the somatic PNS.

In a preferred embodiment, said condition generally involves partial or total loss of function of the nerve, neuron and/or nervous tissue that is damaged.

In a preferred embodiment, the compounds and/or compositions of the invention are suitable in the treatment of PNS injury and/or a degenerative disease of the PNS.

In a preferred embodiment, the compounds and/or compositions of the invention are suitable to and/or capable of:
(1) treating damaged nerves of the PNS after injury, and/or PNS damage other than due to injury, including degenerative disease,
(2) promoting regeneration of PNS nerves after injury and/or PNS damage other than due to injury,
(3) clearing myelin debris around damaged nerves of the PNS,
(4) promoting the regrowth of axons of the PNS after injury or PNS damage other than due to injury,
(5) promoting the recovery of nerves of the PNS after injury and/or PNS damage other than due to injury,
(6) promoting sensory function recovery after PNS injury and/or PNS damage other than due to injury and/or,
(7) promoting the conversion of Schwann cells into the repair phenotype.

PNS damage other than due to injury also includes lesions that are not due to injury, for example.

The invention may achieve one or more of the above effects (1)-(7), including combinations of two or more and even all effects. By way of these effects, the compounds and/or compositions of the invention are generally useful in the treatment of PNS injury and/or a degenerative disease of the PNS.

In some aspects, the present application relates to a new isoform of TRAF7 and to proteins and/or polypeptides comprising the new isoform. The new isoform of TRAF7 is shorter than previously reported isoforms of TRAF7, and thus has a lower molecular weight. In terms of structure, the new isoform lacks one or more WD40 repeat domains, and in some embodiments all WD40 repeat domains, which are usually present in known isoforms of TRAF7.

In an embodiment, the isolated protein of the invention comprises one or more selected from the group of: a RING finger domain, a E3 ubiquitin ligase domain, and a coiled-coil domain. Preferably, the protein further comprises a TRAF-type Zinc finger domain. Preferably, the protein comprises less than 7, 6, 5, 4 complete WD40 repeat domains, preferably less than 3 or 2 WD40 repeat domains and most preferably only one truncated WD40 repeat domain. Preferably, the protein lacks a complete and/or full-length WD40 domain and thus just comprises a truncated WD40 domain, or possibly no WD40 domain at all. It is noted here that reference to any domain recited in this specification is supposed to mean the full-length, complete and/or functional domain, unless otherwise indicated. For example, a truncated WD40 domain is not a complete domain.

The protein comprises an amino acid sequence of SEQ ID NO: 6, or an amino acid sequence having at least 80% sequence identity with SEQ ID NO: 6, preferably at least 85%, 90%, 95%, 96%, 97%, 98%, 99% or 100% sequence identity with SEQ ID NO 6.

Percent identity between two aligned sequences may be determined by the formula: Percent identity = (total number of amino acids - positions of divergent amino acids) / total number of amino acids/ x 100%. It is noted that alignment between two sequences to be compared is made such that sequence identity is maximal. Divergent amino acids include mismatches and gaps.

Besides the amino acid sequence related to short TRAF7, the isolated protein may comprise additional amino acids, oligo- or polypeptides, such as marker peptides, peptides useful for isolation and/or in separation methods, such as tags, in particular affinity-, epitope-, and reporter-based tags, including fusion tags, which may be polypeptides and/or enzymes added to the amino (N) or carboxy (C) terminus of a protein. One of the simplest and smallest tags is the polyhistidine tag, such as the 6XHis tag. Other commonly used tag are the GST, Flag, HA, Halo, HiBiT- and luciferase tags, or tags comprising predetermined amino acid sequences recognized by a specific protease.

Accordingly, the protein of the invention may comprise amino acids, oligo- or polyppetides, such as tags, on either one or both, the N and the C terminus of short TRAF7. One may also envisage additional amino acid residues on the N or C terminus that do not have any particular function and do not affect the biological activity of short TRAF7.

In some embodiments, the protein of the invention may comprise modified amino acid residues, for example short TRAF7 in which a compound is covalently linked to the side chain of one or more amino acids and/or to the free N or C group of the one or more protein and/or the polypeptide with SEQ ID NO: 6.

Short TRAF7 and/or the protein of the invention preferably has a molecular weight of less than 70kDa, less than 68kDa, less than 65kDa, preferably less than 60kDa, even more preferably less than 55kDa, and most preferably about 50kDa, for example from 50-75, 54-68 or 54-56kDa.

Preferably, short TRAF7 is a naturally occurring isoform of TRAF7, and the protein of the invention comprises and/or consists essentially of the naturally occurring short TRAF7, for example having the amino acid sequence of SEQ ID NO:6.

In some aspects, the invention provides antibodies, also known as Immunoglobulins (Ig) or fragments thereof that specifically bind to short TRAF7 and/or the protein of the invention. The antibody of the invention may be monoclonal or polyclonal.

The expression "specifically binds", in the context of the specific binding to short TRAF7, is intended to mean that the antibody binds, under conditions conducive to such specific binding, to short TRAF7, but not to other isoforms of TRAF7, in particular not to the longer isoforms that comprises several WD40 domains as detailed in this specification. The antibody or fragment thereof preferably also does not bind to other, less related proteins than TRAF7. For example, the specific antibody may bind to an epitope of short TRAF7 that would be at least partially covered by WD40 domains of previously reported, long isoforms of TRAF7.

The antibody may be of any class, such as IgG, IgA, IgM, IgE or IgD. Fragments of antibodies preferably comprise one or two binding domains (single, double or multiple domain fragments) (e.g. sdAb) similar to or corresponding to the variable (V) domains, such as V_{L} and V_{H} domains, of antibodies. The fragment may also comprise one or more constant (C) domains, such as constant domains selected from C_{H}1, C_{L}, C_{H}2 and/or C_{H}3 domains. Antibody fragments (F) also include artificial fragments, such as fusion proteins, such as Fv (or scFv, single chain fragments), chemically linked fragments (F(ab')₂) but may generally be further selected from Fab, Fv F(ab'), F(ab')₂, F_{C} (crystallizable), and VHH (variable heavy single domain antibodies or nanobodies) fragments and/or constructs. The invention also encompasses minibodies, diabodies, single chain diabodies, bispecific Fab'2, tandem scFv, to provide a nonexhaustive list.

The antibody or fragment thereof may be human, humanized, chimeric, of animal or human origin, including mouse and rabbit origin.

In some embodiments and/or aspects, the invention relates to methods of screening. The methods of screening may be *in vitro, in vivo, ex vivo,* using living cells, e.g. Schwann cells or any other suitable cell, and/or in solution, without using any living cells. Preferably, for evaluating if a candidate compound affects intracellular levels of HDAC8 and/or short TRAF7, and/or for evaluating if a candidate compound affects intracellular levels of long TRAF7, living cells are used, preferably in an *in vitro* and/or *ex vivo* setting.

Typically, the method of screening comprises using a microtiter plate or any other recipient useful for screening, using the components of an assay that is suitable to generate a convenient read-out of the screened bioactivity (the "desired", "looked for" and/or "screened" (bio)activity), which is generally one of the following:
- reduction of intracellular levels of HDAC8 and/or short TRAF7,
- prevention of the binding of HDAC8 to short TRAF7, and/or
- increase intracellular levels of long TRAF7.

An suitable assay may, for example, detect binding of HDAC8 to short TRAF7 using BRET or FRET mediated read-out. Assays detecting expression of HDAC8 and/or TRAF7 (long or short isoforms) may involve genetically engineering HDAC8 and/or TRAF7 genes to be expressed as fusion proteins, in which the HDAC8 and/or TRAF7 protein is fused to a marker protein, which can be easily detected.

In some embodiments, the assay may use recombinently produced proteins, such as recombinant HDAC8, short and/or long TRAF7, for example, including fusion proteins comprising any one or more selected from HDAC8, short and long TRAF7, or fragments thereof.

The method of screening may involve using a candidate compound. The candidate compound is generally from a library of compounds, and the method of screening has the purpose of determining if the candidate compound (or composition) exhibits one of the desired bioactivities. It is expected that most of the candidate compounds subjected to the screening method of the invention do not have the desired activity, but if a compound is found to have one of the bioactivities, that compound is potentially suitable for treating PNS injury, for treating a degenerative disease of the PNS, and/or for the purpose of one or more selected from (1)-(7) as detailed in this specification. The compound is, however, only "potentially" suitable in the treatment, since other factors, such as toxicity and the therapeutic window are also relevant for determining if a potentially suitable compound can be effectively used in treatment.

While certain of the preferred embodiments of the present invention have been described and specifically exemplified above, it is not intended that the invention be limited to such embodiments. Various modifications may be made thereto without departing from the scope and spirit of the present invention, as set forth in the following claims. Herein below, examples of the invention are disclosed. These examples are for illustration only and are not intended to limit the scope of the present invention.

### Examples

Here, we show that HDAC8, a member of the class I HDAC family of proteins whose functions in SCs remained so far unreported, counteracts the stabilization of HIF1a in SCs, thereby slowing down HIF1a-dependent c-Jun phosphorylation and upregulation, axonal regrowth, and functional recovery after injury. Unexpectedly, we found that HDAC8 regulates this mechanism specifically in SCs ensheathing sensory axons, thereby specifically controlling the regrowth of sensory axons and the recovery of sensory function.

This study complies with all relevant ethical regulations concerning animal use, which was approved by the Veterinary office of the Canton of Fribourg, Switzerland, the Veterinary office (Landesuntersuchungsamt) of Rheinland-Pfalz, Germany, and the Veterinary office (Landesdirektion) in Sachsen, Germany.

Statistical significance is generally obtained by paired or unpaired two-tailed Student's t-tests. In the figures, p values: *<0.05, **<0.01, ***<0.001, values=mean, error bars=s.e.m., with n varying from 3 to 12 depending on the experiment.

### Example 1: Ablation of HDAC8 in adult Schwann cells accelerates myelin clearance, regrowth of sensory axons and sensory function recovery after injury

First, expression of HDAC8 in peripheral nerves after injury is assessed in isoflurane-anesthetised mice from mixed strains backcrossed to C57BL/6 J. Sciatic nerve crush lesions were carried out on three- to four-month-old adult mice (males and females) by a procedure that we have previously described (Brügger, V. et al. Delaying histone deacetylase response to injury accelerates conversion into repair Schwann cells and nerve regeneration. Nat. Commun. 8, 14272 (2017)). Buprenorphine was administered for anesthesia before and after surgery and a carbomer liquid eye gel (Lacryvisc, Alcon, or Viscotears, Novartis) was used preoperatively to prevent dehydration of the eyes.

We collected injured sciatic nerves from the lesion site to around 12 mm distal to the lesion site (region where the nerve splits into the three branches of tibial, sural and common peroneal nerves) and the same region of the contralateral nerve as internal control for each animal. After perineurium removal, sciatic nerves were frozen in liquid nitrogen, pulverized with a chilled mortar and pestle, lysed in radioimmunoprecipitation assay (RIPA) buffer and centrifuged to pellet debris. Supernatants were collected. Sciatic nerve lysates were submitted to SDS-PAGE and analyzed by Western Blotting.

Cytoplasmic and nuclear fractions were obtained by following the protocol described in Senichkin, V. V., Prokhorova, E. A., Zhivotovsky, B. & Kopeina, G. S. Simple and efficient protocol for subcellular fractionation of normal and apoptotic cells. Cells 10, 852 (2021).

We found that after a sciatic nerve crush lesion, HDAC8 expression is upregulated in peripheral nerves distal to the lesion site and is exclusively or in major part found in the cell cytoplasmic compartment, until at least 12 days post lesion (dpl) (results not shown here).

To test for a potential function of HDAC8 in SCs during the regeneration process after lesion, we ablated HDAC8 specifically in adult SCs. To this aim, we crossed *Hdac8* floxed mice with mice expressing the tamoxifen-inducible CreERT2 recombinase under control of the *Plp* promoter (HDAC8 KO) that is active in SCs (and oligodendrocytes) and used *Plp*-CreERT2-negative littermates as control mice. To induce HDAC8 ablation, we injected 2 mg tamoxifen per day for five consecutive days. Control mice received tamoxifen injections simultaneously to HDAC8 KO mice. Mice were submitted to a sciatic nerve crush lesion two weeks after tamoxifen injections, where HDAC8 was efficiently ablated in SCs (result not shown). Surprisingly, the regeneration process after lesion was not impaired by the ablation of HDAC8, but instead was accelerated. Indeed, we found by electron microscopy (images not shown here) that myelin debris were cleared faster and more axons per surface area, including Remak axons, had regrown at 12 dpl (**Figure 1**) in HDAC8 KO compared to control mice.

To test for a potential improvement in functional recovery, we crossed *Hdac8* floxed mice with mice expressing the tamoxifen-inducible CreERT2 recombinase under control of the *P0* promoter (also called thereafter HDAC8 KO, only used for functional recovery experiments) that is active only in SCs and used *P0*-CreERT2-negative littermates as control mice.

To do so, mice homozygous for floxed *Hdac8* allele (EMMA, Strain # EM:05717) were crossed with *P0*-CreERT2 or Plp-CreERT2 mice, in which the expression of the Cre recombinase (Cre) is tamoxifen-inducible and controlled by the Schwann cell-specific *P0* promoter or the Schwann cell and oligodendrocyte-specific *Plp* promoter. Mice homozygous for floxed *Hif1α* allele (The Jackson Laboratory, Strain # 007561), were crossed with *Plp-*CreERT2 mice. To ablate HDAC8 or HIF1α in adult SCs, three- to four-month-old adult mice received daily injections of 2 mg tamoxifen (Sigma) for five consecutive days. As control mice, Cre-negative littermates of HDAC8 or HIF1α knockout mice were used. Genotypes were determined by PCR on genomic DNA.

Here again, mice were submitted to a sciatic nerve crush lesion two weeks after tamoxifen injections, where HDAC8 was also efficiently ablated in SCs (result not shown).

Recovery of motor function was tested at 12, 19 and 30 dpl using the Rotarod test and the inverted grid test. For these tests, mice were exposed to the protocols set out in Duman, M. et al. "EEF1A1 deacetylation enables transcriptional activation of remyelination" Nat. Commun. 11, 3420 (2020) and in Duman, M. et al. "Theophylline induces remyelination and functional recovery in a mouse model of peripheral neuropathy". Biomedicines 10, 1418 (2022). For both tests, mice were first trained three times and trials were separated by a 30 min recovery period. Recovery of sensory function was tested at 12, 19 and 30 dpl by toe pinch test: each toe of the rear foot on the right side (lesioned side) was pinched with equal pressure applied by the same experimenter using flat tip forceps. Immediate withdrawal was recorded as functional sensitivity of the pinched toe. In case no toe exhibited sensitivity, the same test was applied to toes of the contralateral side (uninjured side), which always resulted in immediate withdrawal.

We found that sensory function recovery was faster in HDAC8 KO mice (**Figure 2**). However, motor function recovery and remyelination (not shown) were comparable in both HDAC8 KO and control mice and we did not detect compensatory expression of HDAC1, HDAC2 or HDAC3 (not shown), the other members of class I HDACs.

We then carried out whole-nerve stainings for Stathmin-2 (STMN2, also called SCG10) and growth-associated protein 43 (GAP43) in sciatic nerves of HDAC8 KO and control mice at 3 dpl. STMN2 is a highly selective marker of regrowing axons that preferentially labels sensory axons after sciatic nerve injury, while GAP43 is a general marker of regrowing axons. Consistent with faster sensory function recovery, we found that STMN2-labeled axons had regrown over a longer distance at 3 dpl in sciatic nerves of HDAC8 KO mice compared to control mice (image not shown, **Figure 3**), whereas we could not detect a significant difference for GAP43-labeled axons (not shown).

We thus asked whether HDAC8 is specifically expressed in SCs associated with sensory axons, which could potentially explain why HDAC8 ablation has a specific effect on the regrowth and functional recovery of sensory axons. To answer this question, we co-stained HDAC8 with STMN2 that preferentially labels sensory axons and Neurofilament that labels all axons. Indeed, SCs expressing high levels of HDAC8 were associated with STMN2-positive axons, whereas SCs associated with STMN2-negative axons (Neurofilament-positive) did not express HDAC8 or at very low level (**Figure 4**). Of note, HDAC8 was also expressed in some axons (not shown).

Taken together, our results identify HDAC8 as a specific marker of SCs associated with sensory axons in adult sciatic nerves and show that the ablation of HDAC8 in SCs promotes myelin debris clearance, regrowth of sensory axons and sensory function recovery.

### Example 2: HDAC8 ablation in Schwann cells enhances hypoxia-induced c-Jun phosphorylation and upregulation

Based on quantification of Western blots of crushed and collateral (non-crushed controls) sciatic nerves, we found that the levels of c-Jun (**Figures 5A and 5B**) and phosphorylated c-Jun (not shown) were increased in SCs of HDAC8 KO compared to control mice, already at 1 dpl. C-Jun expression levels remained increased in SCs of HDAC8 KO mice until at least 3 dpl and returned to control levels at 5 dpl (5 and 12 dpl not shown. Oct6 expression levels in SCs were not affected by the ablation of HDAC8 (result not shown), indicating that the mechanisms underlying c-Jun upregulation in HDAC8 KO SCs are different than the mechanisms responsible for c-Jun upregulation in HDAC1/2 KO SCs.

To elucidate these mechanisms, we used purified primary rat SCs cultured under conditions that mimic the conversion into the repair SC phenotype occurring after a peripheral nerve lesion, where myelin proteins are downregulated and c-Jun upregulated.

Purified primary rat SC cultures were derived from P2 Wistar rat sciatic nerves, as previously described in Jacob, C. et al. 2Expression and localization of Ski determine cell type-specific TGFbeta signaling effects on the cell cycle". J. Cell Biol. 182, 519-530 (2008). SCs were then purified by sequential immunopanning in plastic dishes coated with a Thy1.1 antibody. Identity and purity were checked for each primary preparation by immunofluorescence of SC-specific markers (Sox10, Oct6, Krox20, P0, MAG). SCs were grown in DMEM proliferation medium. SC culture protocol mimicking SC demyelination and conversion into repair cells that occur after a PNS lesion was as described in Brügger, V. et al. "Delaying histone deacetylase response to injury accelerates conversion into repair Schwann cells and nerve regeneration". Nat. Commun. 8, 14272 (2017).

To downregulate HDAC8 in primary SCs, we used a lentiviral vector carrying a highly efficient HDAC8-specific shRNA (result not shown).

Generally, to produce lentiviral particles, HEK293T cells (ATCC) were co-transfected with each lentiviral construct: HDAC8 shRNA (TRCN00000877999), TRAF7 shRNA (TRCN0000302734), HIF1a shRNA (TRCN0000232221) or control shRNA (SHC001), together with the packaging constructs pLP1, pLP2 and pLP/VSVG (Invitrogen), using Lipofectamine 2000 (Invitrogen), according to the recommendations of the manufacturer (ViraPower Lentiviral Expression Systems Manual). Lentiviruses were added to primary rat SCs in defined medium (DM) and incubated for two days. Then, infected cells were selected by adding 2 µg/ml puromycin (Sigma) in DM for two days, before adding proliferating medium. HDAC8 shRNA, TRAF7 shRNA, HIF1-α shRNA and control shRNA lentiviral constructs were purchased from Sigma-Aldrich.

Transfection: Confluent primary rat SCs were transfected either in differentiating conditions or in conditions mimicking the conversion into the repair phenotype at the time of change to proliferation medium with Fugene 6 (Promega) at 5:1 ratio Fugene 6:DNA, according to the manufacturer's recommendations, with minor modifications.

HDAC8 downregulation in SCs led to increased levels of c-Jun and phosphorylated c-Jun compared to levels in SCs transduced with a lentiviral vector carrying a non-targeting control shRNA (not shown), an effect that was however more significant under hypoxic conditions, either by incubation in a hypoxia chamber (**Figures 5C** and **5D**) or incubation with cobalt chloride (CoCl₂) which mimics hypoxic conditions (not shown).

Hypoxia is known as a robust inducer of c-Jun expression and phosphorylation and occurs after a peripheral nerve injury. Additionally, we found that knocking out HIF1α specifically in adult SCs by crossing *Plp*-CreERT2 mice with *Hif1α*-floxed mice and inducing *Hif1α* recombination by tamoxifen injections, such as described above for *Hdac8* recombination, leads to reduced c-Jun upregulation after lesion (not shown). For these reasons, we carried out our cell cultures under hypoxia and tested whether hypoxia is involved in HDAC8-mediated regulation of c-Jun expression and phosphorylation after injury. In addition to increased c-Jun protein levels, we found that HDAC8 downregulation in primary SCs led to increased *c-Jun* mRNA levels under hypoxic conditions (**Figure 5E**), but only to a trend under normoxia (not shown). Consistently, HDAC8 downregulation resulted in increased activation of the *c-Jun* promoter under hypoxic conditions (**Figure 5F**), but not under normoxia (not shown). These results indicate that HDAC8 counteracts c-Jun upregulation and phosphorylation, in particular under hypoxic conditions.

Hypoxia leads to the stabilization of HIF1α and its translocation to the nuclear compartment (not shown). Consistent with HDAC8 cytoplasmic localization in contralateral and crushed sciatic nerves of adult mice (see above), we found that HDAC8 is exclusively localized in the cytoplasm of primary rat SCs cultured under conditions mimicking the conversion into the repair phenotype in normoxia or hypoxia (**Figure 6A**). HDAC8 downregulation increased HIF1α levels under normoxic and hypoxic conditions (**Figures 6B** and **6C**), and HIF1α downregulation by shRNA (not shown) reduced c-Jun expression and phosphorylation (not shown).

We hypothesized that HDAC8 promotes HIF1α degradation. Indeed, when we inhibited the proteasome by a treatment with the proteasome inhibitor MG-132 (20 µM MG-132 (proteasome inhibitor, Hycultec) or its vehicle were added to rat SCs in conditions mimicking the conversion into the repair phenotype for 4 to 8 h.), HIF1α levels were no longer increased in cells where HDAC8 was downregulated compared to controls (**Figure 6D**). Taken together, these data indicate that the increased levels of HIF1α in SCs mediated by HDAC8 downregulation are due to the protection of HIF1α from proteasomal degradation.

Under normoxia, the Von Hippel-Lindau (VHL) tumor suppressor interacts with HIF1α and recruits a protein complex with E3 ubiquitin ligase activity that induces HIF1α ubiquitination and subsequent targeting to the proteasome. However, expression levels of VHL were increased in normoxic conditions in SCs where HDAC8 was downregulated by shRNA (not shown), probably to compensate the increased levels of HIF1α, as previously shown, suggesting that HDAC8 regulates HIF1α stability by a VHL-independent mechanism. Consistent with our cell culture findings, we show that HIF1α levels are increased in sciatic nerves of HDAC8 KO mice compared to control mice already at 1 dpl (not shown), indicating that HDAC8 KO SCs can rapidly upregulate HIF1α after injury. Interestingly, we identified this early increase of HIF1α levels in the cytoplasmic compartment, while nuclear levels of HIF1α were not affected (not shown), indicating that HIF1α-dependent regulation of c-Jun expression and phosphorylation at 1dpl is mediated by a mechanism occurring in the cytoplasm. Similarly, at an early time point of hypoxia in rat SCs, the increase of HIF1α levels due to HDAC8 downregulation occurred in the cytoplasm (not shown), whereas no HIF1α was detected in the nucleus (data not shown). Several studies have also reported high levels of HIF1α in the cytoplasmic compartment while nuclear levels remained low, suggesting a function of HIF1α in the cytoplasm. C-Jun is phosphorylated by JNK and in turn phosphorylated c-Jun dimerizes with ATF-2 to activate c-Jun expression. In addition, the JNK pathway is known to mediate various hypoxia-induced cellular processes. We found that in SCs cultured under hypoxic conditions, HIF1α downregulation by shRNA led to decreased activation of JNK (not shown), and JNK inhibition (1 µM JNK-IN-8 (JNK inhibitor, MedChemExpress)) completely abrogated the increase of c-Jun phosphorylation and partly prevented c-Jun upregulation mediated by HDAC8 downregulation (not shown). Taken together, our data indicate that the ablation or downregulation of HDAC8 in SCs leads to HIF1α- and JNK-dependent c-Jun phosphorylation and upregulation.

### Example 3: HDAC8 regulates TRAF7 expression to promote HIF1α degradation and to prevent c-Jun phosphorylation

To understand how HDAC8 regulates HIF1a stabilization, we first asked whether HDAC8 deacetylase activity is involved in this function. We tested 2 different HDAC8 specific inhibitors, PCI-34051 and 1-Naphthohydroxamic acid, at different concentrations. None of the two HDAC8 inhibitors was able to increase HIF1a levels, c-Jun expression or phosphorylated c-Jun levels in SCs (not shown), indicating that these effects are independent of HDAC8 deacetylase activity.

HDAC8 has been previously shown to have in some instances deacetylase-independent functions, including acting as a scaffold for protein complexes or preventing the degradation of its binding partners. To identify HDAC8 putative binding partners, we immunoprecipitated HDAC8 in unlesioned mouse sciatic nerves and at 1 day post sciatic nerve crush lesion, and used a non-targeting IgG as immunoprecipitation control. We then analyzed co-immunoprecipitated proteins by mass spectrometry. We found 9 interesting, significantly enriched terms among which "Ubiquitin conjugation" showed the highest number of matching proteins (not shown). Among the putative binding partners of HDAC8 with a function in ubiquitin conjugation, we focused on tumor necrosis factor receptor-associated factor 7 (TRAF7) that is localized in the cytoplasmic compartment in both primary rat SCs (**Figure 7A**, left panel) and adult sciatic nerves (**Figure 7A**, right panel) such as HDAC8, possesses a RING domain with E3 ligase activity and can therefore mediate ubiquitination.

Our mass spectrometry analyses indicated TRAF7 as a putative binding partner of HDAC8 in both unlesioned nerves and nerves at 1 day post sciatic nerve crush lesion (not shown).

We first confirmed that TRAF7 co-immunoprecipitated with HDAC8 in primary rat SCs (**Figure 7B**).

We used two different TRAF7 antibodies, which both detected three to four bands in primary rat SCs and in mouse sciatic nerves, two-three ranging from around 65 to 75 kDa and one shorter around 50 kDa (Figures 7A, 7B). Multiple isoforms of TRAF7 ranging from 65 to 75 kDa are commonly described. The NCBI database describes the transcript (accession number: XR_007064922.1) of a predicted TRAF7 variant that has not been reported yet, but that would contain the RING finger domain with E3 ubiquitin ligase activity, the TRAF-type Zinc finger domain and the coiled-coil domain, but only one truncated WD40 repeat domain instead of the 7 WD40 repeat domains found in the longest TRAF7 isoform (Zotti, T. et al. "The emerging role of TRAF7 in tumor development". J. Cell. Physiol. 232, 1233-1238 (2017)). To test whether all bands detected by the TRAF7 antibodies are isoforms of TRAF7, we downregulated TRAF7 in primary rat SCs by using a lentivirus carrying a specific TRAF7 shRNA targeting a sequence common to all TRAF7 isoforms. Indeed, all four bands were downregulated (**Figure 7C**), indicating that they correspond to four different isoforms of TRAF7. Among these four isoforms, we found that the shorter isoform co-immunoprecipitated with HDAC8 (**Figure 7B**).

Interestingly, in primary rat SCs where HDAC8 was downregulated by shRNA, expression of the shorter TRAF7 isoform was significantly decreased while expression of the longest isoform was increased (**Figures 7D** and **7E**). In sciatic nerves, we found that TRAF7 is expressed in all SCs (**Figure 7F**), while as described above, HDAC8 is present only in sensory SCs and in axons. Consistent with our cell culture findings, short TRAF7 was downregulated in HDAC8 KO nerves, while long TRAF7 was upregulated (**Figure 7G**). Of note, short TRAF7 was also downregulated in crushed nerves of Control mice at 1 dpl compared to contralateral nerves, but not further downregulated in crushed nerves of HDAC8 KO mice compared to their contralateral nerves (**Figures 7G** and **7H**). This suggests that short TRAF7 is completely downregulated already in SCs of contralateral nerves of HDAC8 KO mice and can therefore not be further downregulated. The remaining expression of short TRAF7 is thus likely to be due to expression in non-sensory SCs. These results indicate that HDAC8 differentially regulates TRAF7 isoforms by stabilizing the short isoform and in contrast destabilizing the long isoform.

We then asked whether TRAF7 is involved in HDAC8-dependent degradation of HIF1a and subsequent effects on c-Jun regulation. We found that TRAF7 downregulation by shRNA in primary rat SCs led to increased HIF1a levels in normoxic conditions (**Figure 7I**) and increased c-Jun levels in hypoxic conditions (**Figure 7J**), similar to the effects of HDAC8 downregulation. In addition, by co-immunoprecipitation we show that short TRAF7 interacts with HIF1a in primary rat SCs (**Figure 7K**) and that HIF1a ubiquitination is decreased in SCs where TRAF7 is downregulated by shRNA (**Figure 7L**).

However, in contrast to HDAC8 downregulation, we found that TRAF7 downregulation led to decreased levels of phosphorylated c-Jun in normoxia and has no effect on HIF1a and phospho-c-Jun levels in hypoxia (not shown). It has been previously shown that TRAF7 can promote JNK phosphorylation, which in turn increases HIF1a levels and phosphorylates c-Jun. Indeed, we show in primary rat SCs that TRAF7 downregulation leads to decreased phospho-JNK levels in normoxia but not in hypoxia (not shown). It is highly likely that this effect is due to the downregulation of the long TRAF7 isoforms that contain WD40 repeats because TRAF7 is known to promote JNK phosphorylation by interacting with MEKK3 through its WD40 repeat domains. As mentioned above, short TRAF7 contains only one truncated WD40 repeat domain and it was shown that a mutant of TRAF7 missing the WD40 repeat domains cannot interact with MEKK3 and that long TRAF7 cannot promote JNK phosphorylation in the absence of MEKK3. Since HDAC8 stabilizes short TRAF7 but destabilizes long TRAF7 (Figs 7D and 7E), we conclude that HDAC8 downregulation leads through the upregulation of long TRAF7 to increased phospho-JNK levels, inducing increased phospho-c-Jun levels and consequently increased c-Jun levels, and through the downregulation of short TRAF7 to increased HIF1a levels, resulting in JNK-dependent increase of phospho-c-Jun and c-Jun levels and to JNK-independent increase of c-Jun levels. Consistently, we show here that overexpression of TRAF7 (long and short isoforms) reverted the increase of HIF1a and c-Jun but not of phospho-c-Jun levels mediated by HDAC8 downregulation through shRNA (not shown). Of note, all four TRAF7 isoforms were overexpressed by transfection of a construct carrying the longest isoform, indicating that the isoforms detected by Western blot result either from post-translational modifications of the long isoform or potentially also from mRNA heterosplicing, a mechanism that has been recently described.

We then obtained additional data on short TRAF7 by a cloning strategy starting from human long TRAF7 using 3 couples of primers to obtain short TRAF7 cDNA, and adding it to a cloning vector. We sequenced the cDNA and obtained the nucleotide and amino acid sequences of the short isoform as shown in **Figure 8** (SEQ ID NO: 5 and 6).

We transfected lysates of rat SCs with a construct expressing short TRAF7 (T7-small) or with a Flag-expressing construct as control (Ctrl), and made TRAF7 western blot (**Figure 9A**), which shows that the short 50-kDa TRAF7 is overexpressed and also reveals a heavier short isoform at around 65 kDa, resulting most likely from posttranslational modification of 50-kDa short TRAF. In the quantification, the two short isoforms (50- and 65-kDa) and the two long isoforms (above 75 kDa) are quantified separately.

To investigate the effect of short TRAF7 in the regulation of HIF1α, c-Jun and p-c-Jun levels, we incubated rat SCs with lentiviruses carrying an HDAC8 shRNA (H8sh) or a control shRNA (Csh) and subsequently transfected them with a short TRAF7-overexpressing construct (T7-small) or a Flag-expressing construct as control (Ctrl). **Figure 9B** shows the results for p-c-Jun levels only. In all experiments, the increase of HIF1α, c-Jun and p-c-Jun mediated by HDAC8 downregulation was reverted.

Taken together, these data show that HDAC8 oppositely regulates the short and the long TRAF7 isoforms, which leads to TRAF7-induced ubiquitination and degradation of HIF1a and slows down JNK phosphorylation, c-Jun phosphorylation and c-Jun upregulation in hypoxic conditions (**Figure 9**).

### Conclusions of Examples 1-3

Ablating HDAC8 specifically in adult SCs before a sciatic nerve crush lesion surprisingly did not impair regeneration, but instead accelerated it, indicating a negative effect of HDAC8 on the regeneration process and demonstrating that this process is not optimal and can be improved. Interestingly, we found that HDAC8 ablation specifically promotes the regrowth of sensory axons and sensory function recovery. Indeed, we show that HDAC8 is exclusively expressed or expressed at high levels in SCs associated with sensory axons and that ablation of HDAC8 leads to increased levels of c-Jun and phosphorylated c-Jun in SCs. These findings show that the conversion into repair SCs is controlled by different mechanisms in sensory and motor SCs and identify HDAC8 as a marker of adult sensory SCs.

By mass spectrometry analysis, we identified TRAF7 as a binding partner of HDAC8. We also showed that HDAC8 and TRAF7 are both exclusively or in large majority localized in the cytoplasmic compartment of SCs present in adult mouse sciatic nerves and in primary rat SCs in culture. By co-immunoprecipitation, we found that short TRAF7, that had not been studied so far but is described as a predicted isoform in the NCBI database, interacts with HDAC8 in SCs *in vivo* and in culture and mediates HDAC8-dependent degradation of HIF1α. TRAF7 possesses a RING finger domain with E3 ubiquitin ligase activity that is capable of catalyzing protein ubiquitination and thereby targeting proteins to the proteasome for degradation. The short 50-kDa TRAF7 isoform misses the C-terminal WD40 repeat domains found in the long TRAF7 isoforms. This structural difference confers to the short isoform different functions compared to the long TRAF7 isoforms regarding the activation of JNK. Indeed, TRAF7 is known to promote JNK phosphorylation and subsequent c-Jun phosphorylation by binding to MEKK3 through its WD40 repeat domains. Short TRAF7 missing these domains does not have this function. Instead, this isoform is stabilized, most likely through its interaction with HDAC8, and induces HIF1α ubiquitination and subsequent HIF1α degradation. Ablation of HDAC8 prevents this mechanism by leading to the downregulation of short TRAF7 and instead results in increased HIF1α levels and HIF1α-mediated c-Jun phosphorylation and upregulation. In addition, we show that ablation of HDAC8 leads to the upregulation of long TRAF7, which potentiates JNK phosphorylation and thereby c-Jun phosphorylation and upregulation.

In summary, our study identifies a pathway controlled by HDAC8 that modulates HIF1α stabilization and JNK activation, and subsequent c-Jun phosphorylation and expression specifically in sensory SCs. This function of HDAC8 is not linked to its deacetylase activity, therefore HDAC8 inhibitors cannot be used to promote the regrowth of sensory axons and sensory function recovery. Instead, HDAC8 should be downregulated, either by using RNA interference or by repressing *Hdac8* gene activation. Alternatively, TRAF7 inhibitors or HIF prolyl hydroxylase inhibitors that enhance HIF1α stabilization could also be tested to promote regeneration.

## Claims

1. A compound and/or a composition suitable to:
- reduce intracellular levels of HDAC8 and/or short isoform TRAF7,
- prevent the binding of HDAC8 to short isoform TRAF7, and/or
- increase intracellular levels of long isoform TRAF7,
for use in the treatment of PNS injury and/or a degenerative disease of the PNS.

2. A compound and/or a composition suitable to:
- reduce intracellular levels of HDAC8 and/or short isoform TRAF7,
- prevent the binding of HDAC8 to short isoform TRAF7, and/or
- increase intracellular levels of long isoform TRAF7,
for use in one or more selected from:
(1) treating damaged nerves of the PNS after injury or PNS damage other than due to injury, including degenerative disease,
(2) promoting regeneration of PNS nerves after injury or PNS damage other than due to injury,
(3) clearing myelin debris around damaged nerves of the PNS,
(4) promoting the regrowth of axons of the PNS after injury or PNS damage other than due to injury,
(5) promoting the recovery of nerves of the PNS after injury or PNS damage other than due to injury,
(6) promoting sensory function recovery after PNS injury or PNS damage other than due to injury, and/or,
(7) promoting the conversion of Schwann cells into the repair phenotype.

3. The compound and/or a composition for use of any one of the preceding claims, which reduces intracellular levels of HDAC8 and/or short isoform TRAF7 by one or more selected from:
(a) repression of gene activation and/or expression of HDAC8 and/or short isoform TRAF7,
(b) downregulation of HDAC8 and/or short isoform TRAF7,
(c) otherwise reduction of intracellular levels of HDAC8 and/or short isoform TRAF7, and/or which increases intracellular levels of long isoform TRAF7 by one or more selected from:
(d) upregulation of long isoform TRAF7,
(e) inducing the expression of long isoform TRAF7, and/or
(f) otherwise increase intracellular levels of long isoform TRAF7.

4. The compound and/or composition for use of any one of the preceding claims, wherein said PNS is specifically the somatic nervous system of the PNS.

5. The compound and/or composition for use of any one of the preceding claims, wherein said PNS is specifically the sensory PNS and/or wherein nerves of said PNS are specifically sensory nerves.

6. The compound and/or composition for use of any one of the preceding claims, wherein
- reduction of intracellular levels of HDAC8 and/or short isoform TRAF7,
- prevention of the binding of HDAC8 to short isoform TRAF7, and/or
- increase of the intracellular levels of long isoform TRAF7,
is and/or takes place in Schwann cells (SCs), preferably in the cytoplasm of said SCs.

7. The compound and/or composition for use of any one of the preceding claims, wherein said short isoform TRAF7 is the isoform having the amino acid sequence of SEQ ID NO: 6, encoded by the nucleotide sequence of SEQ ID NO: 5, which isoform has a molecular weight of less than 60kDa, preferably less than 55kDa.

8. The compound and/or composition for use of any one of the preceding claims, wherein said short isoform TRAF7 is the isoform comprising a RING finger domain, E3 ubiquitin ligase activity, TRAF-type Zinc finger domain, a coiled-coil domain, and less than 4 complete WD40 repeat domains, preferably only one truncated WD40 repeat domain or no WD40 repeat domain.

9. The compound and/or composition for use of any one of the preceding claims, wherein said long isoform TRAF7 is the isoform encoded by the nucleotide sequence NM_032271, which isoform has a molecular weight of higher than 55kDa, preferably higher than 65kDa, and most preferably 75kDa or higher.

10. The compound and/or composition for use of any one of the preceding claims, wherein said long isoform TRAF7 comprises a RING finger domain, E3 ubiquitin ligase activity, TRAF-type Zinc finger domain, a coiled-coil domain, and 4 or more, preferably 7 complete WD40 repeat domains.

11. The compound and/or composition for use of any one of the preceding claims, wherein said compound and/or composition does not substantially affect, preferably not substantially inhibit, the enzymatic deacetylase activity of HDAC8.

12. The compound and/or composition for use of any one of the preceding claims, wherein said compound and/or composition is or comprises interfering RNA (RNAi), such as an siRNA duplex or a short hairpin RNA (shRNA) or a nucleotide sequence complementary to shRNA and/or suitable to generate an shRNA molecule or an siRNA duplex *in vivo.*

13. The compound and/or composition for use of any one of the preceding claims, wherein said compound and/or composition is or comprises a repressor of HDAC8 or a nucleotide sequence encoding a repressor of HDAC8.

14. The compound and/or composition for use of any one of the preceding claims, wherein said compound and/or composition is administered by transdermal administration and/or in the form of a subcutaneous depot, preferably at the site of injury and/or at the site of the damaged nerve of the PNS.

15. An isolated protein comprising the amino acid sequence of SEQ ID NO: 6, or an amino acid sequence having at least 80% sequence identity with SEQ ID NO: 6, wherein said protein comprising a RING finger domain, E3 ubiquitin ligase activity, TRAF-type Zinc finger domain, a coiled-coil domain, and less than 4 complete WD40 repeat domains, preferably less than 3 or 2 WD40 repeat domains and more preferably only one truncated WD40 repeat domain or no WD40 repeat domain.

16. The isolated protein of claim 15, wherein said amino acid sequence is the amino acid sequence of short isoform TRAF7.

17. An antibody that specifically binds to the isolated protein of any one of claims 15 and 16, and which antibody does not bind to isoforms of TRAF7 that comprise 4 or more WD40 domains.

18. A method for screening for compounds and/or compositions potentially suitable in the treatment of injury to PNS nerves, wherein said method comprises determining, whether a candidate compound and/or composition is suitable to one or more selected from:
- reduce intracellular levels of HDAC8 and/or short isoform TRAF7,
- prevent the binding of HDAC8 to short isoform TRAF7, and/or
- increase intracellular levels of long isoform TRAF7.
